# EUROPEAN PATENT APPLICATION

(11) **EP 4 772 609 A1**
(43) Date of publication of application: **08.07.2026**
(21) Application number: 24860424.1
(22) Date of filing: 29.08.2024
(51) Int. Cl.: C12N 1/20, A61K 35/745, A61K 35/747, A61P 21/00, A61P 25/28, A61P 25/14, A61P 25/16, A61P 29/00, A23L 33/135, C12R 1/01

(54) **NOVEL LACTIC ACID BACTERIA AND USE THEREOF**

(30) Priority: 01.09.2023 KR 20230116398
(71) Applicant: Dong Wha Pharm. Co., Ltd., Seoul 04516 (KR); Pblbiolab Co., Ltd., Seoul 02823 (KR)
(72) Inventor: HWANG, Yun ha, Gunpo-si, Gyeonggi-do 15822 (KR); OH, Seong Jun, Yongin-si, Gyeonggi-do 16875 (KR); WON, Dae Yeon, Yongin-si, Gyeonggi-do 17066 (KR); KIM, Su Bin, Suwon-si, Gyeonggi-do 16707 (KR); KIM, Dong-Hyun, Seoul 02823 (KR)
(74) Representative: Cabinet Becker et Associés
(86) International application number: PCT/KR2024/012929
(87) International publication number: WO 2025/048505

(57) **Abstract**

The present disclosure relates to novel *lactic* acid bacteria *Bifidobacterium bifidum* P61 KCCM13367P, *Lactobacillus paracasei* P62 KCCM13368P, or a mixture thereof.

The novel lactic acid bacteria of the present disclosure and the mixture thereof alleviate sarcopenia by changing the intestinal microbial composition to inhibit the expression of sarcopenia markers (MuRF-1, MAFbx/Atrogin-1, etc.), increase muscle strength and muscle mass, and promote muscle formation. In addition, they ameliorate cognitive impairment by inhibiting behaviors similar to cognitive impairment and regulating the expression of factors associated with the impairment (BDNF, IL-10, etc.). Furthermore, they have the effect of treating inflammation by reducing the expression of inflammatory markers (TNF-α, IL-6, IL-1β and MPO) and increasing the expression of anti-inflammatory markers (IL-10).

## Description

### Technical Field

The present disclosure relates to novel lactic acid *bacteria, Bifidobacterium bifidum* P61 KCCM13367P, *Lactobacillus paracasei* P62 KCCM13368P, or a mixture thereof, and a use thereof.

### Background Art

As the world enters an aging society, people are exposed to various diseases, and accordingly, the importance of aging-related diseases is being emphasized. The most representative physiological change due to aging is a decrease in muscle mass and muscle strength. The human body is composed of about 600 muscles, and these muscles account for half of the body weight. Muscle is formed by tens of thousands of myofibers (muscle cells) gathering together, and the myofibers grow in size as they develop, but as they age, their number decreases and their function gradually deteriorates.

Sarcopenia is a disease in which muscle mass continuously decreases as muscle protein degradation occurs more than protein synthesis. This is mainly caused by decreased physical activity, malnutrition, environmental factors, diseases, inflammation, mitochondrial abnormalities, hormonal changes, etc., and research results show that genetic differences also exist. The sarcopenia is a metabolic disease that directly causes a decrease in muscle strength, thereby not only increasing the risk of death due to decreased and impaired various body functions, but also causing an increase in the prevalence of metabolic diseases such as hypertension, diabetes, arthritis, obesity, cancer, etc. due to decreased metabolism and reduced immunity.

Another representative disease caused by population aging is cognitive impairment. The cognitive impairment is a disorder that exhibits cognitive damage and behavioral changes, and refers to diseases arising from functional decline in memory, spatial perception, judgment, executive function, language ability, etc. Dementia, which is related to cognitive decline, was known as a geriatric disease, but due to aging, it has become a disease that anyone may suffer from, and has become a social problem due to the burden of family care and economic burden.

Meanwhile, probiotics are live microbial agents that are distributed as dominant bacteria in the human intestine where various microorganisms exist and promote the growth of beneficial bacteria in the body, and play a role in symbiosis with the human digestive system to decompose fiber and complex proteins into important nutritional components. In addition, they inhibit the proliferation of harmful bacteria such as Escherichia coli and *Clostridium difficile,* ameliorate diarrhea and constipation, and play roles in vitamin synthesis, blood cholesterol reduction, etc. Among these probiotics, lactic acid bacteria are bacteria that ferment sugars to obtain energy and produce large amounts of lactic acid. They are widely distributed in nature, including agricultural products, foods, and the bodies of humans and animals. They are widely used in the fermentation of cheese, fermented milk, kimchi, bread making, etc. Generally, when lactic acid bacteria are consumed, it is known that not only harmful bacteria among intestinal microorganisms are inhibited, but also beneficial bacteria that help digest, absorb, and decompose food increase. Therefore, it has been reported that consuming lactic acid bacteria has various effects such as blood cholesterol level reduction, immunity enhancement, endogenous infection inhibition, liver cirrhosis amelioration, anticancer effects, etc. In addition, research on enhancing the efficacy of natural products fermented with lactic acid bacteria has recently been conducted.

Accordingly, the present inventors have completed the present disclosure by identifying novel lactic acid bacterial strains that exhibit amelioration effects on sarcopenia, cognitive impairment, and inflammatory diseases.

### Summary of Invention

An object of the present disclosure is to provide *Bifidobacterium bifidum* P61 KCCM13367P.

Another object of the present disclosure is to provide *Lactobacillus paracasei* P62 KCCM 13368P.

Another object of the present disclosure is to provide a composition for preventing, treating, or ameliorating sarcopenia, cognitive impairment, and inflammatory diseases, comprising *Bifidobacterium bifidum* P61 KCCM13367P, *Lactobacillus paracasei* P62 KCCM13368P, or a mixture thereof.

In the following description, to avoid confusion due to overlapping content, the description of overlapping content will be omitted. That is, the content of the invention is not limited to the following content alone, but the content of the invention should be interpreted according to the overall content of the invention.

Hereinafter, the present disclosure will be described in detail.

The present disclosure provides *Bifidobacterium bifidum* P61 (depositary Institution: Korean Culture Center of Microorganisms, date of deposit: July 12, 2023, accession number: KCCM13367P).

The *Bifidobacterium bifidum* P61 of the present disclosure is characterized in that it is a lactic acid bacterium isolated and identified from the intestinal microorganisms of a healthy person.

The 16S rDNA sequence for identification and classification of the *Bifidobacterium bifidum* P61 of the present disclosure is the same as SEQ ID NO: 1 attached to this specification. Therefore, the *Bifidobacterium bifidum* P61 of the present disclosure may include the 16S rDNA of SEQ ID NO: 1 as follows:

16S rDNA of Bifidobacterium bifidum P61 (SEQ ID NO: 1):

TACGACTTAGTCCCAATCACGAGCCTCACCTTAGACGGCTCCATCCCACAAGGGGT
TAGGCCACCGGCTTCGGGTGCTGCCCACTTTCATGACTTGACGGGCGGTGTGTAC
AAGGCCCGGGAACGCATTCACCGCGGCGTTGCTGATCCGCGATTACTAGCGACTC

CGCCTTCACGGAGCCGGTTGCAGGCTCCGATCCGAACTGAGACCGGTTTTCAGGG
ATCCGCTCCATGTCGCCATGTCGCATCCCGCTGTACCGGCCATTGTAGCATGCGTG
AAGCCCTGGACGTAAGGGGCATGATGATCTGACGTCATCCCCACCTTCCTCCGAGT
TAACCCCGGCGGTCCCCCGTGAGTTCCCACCATAACGTGCTGGCAACACGGGGCG
AGGGTTGCGCTCGTTGCGGGACTTAACCCAACATCTCACGACACGAGCTGACGAC
GACCATGCACCACCTGTGAACCCGCCCCGAAGGGAAACGCCATCTCTGGCGTCGT
CGGGAACATGTCAAGCCCAGGTAAGGTTCTTCGCGTTGCATCGAATTAATCCGCAT
GCTCCGCCGCTTGTGCGGGCCCCCGTCAATTTCTTTGAGTTTTAGCCTTGCGGCC
GTACTCCCCAGGCGGGACGCTTAACGCGTTAGCTCCGACACGGAACACGTGGAAC
GTGCCCCACATCCAGCGTCCACCGTTTACGGCGTGGACTACCAGGGTATCTAATCC
TGTTCGCTCCCCACGCTTTCGCTCCTCAGCGTCAGTGACGGCCCAGAGACCTGCC
TTCG

As a result of analysis of the 16S rDNA nucleotide sequence of SEQ ID NO: 1, it showed 99% homology with known *Bifidobacterium bifidum* strains (homology with *Bifidobacterium bifidum* KCTC3202 strain), and whole genome analysis also showed 98.8% homology (homology with *Bifidobacterium bifidum* JCM1255 strain), thus showing the highest molecular phylogenetic relationship with *Bifidobacterium bifidum.* Therefore, the lactic acid bacterium was identified as *Bifidobacterium bifidum* and named *Bifidobacterium bifidum* P61, and was deposited with the Korean Culture Center of Microorganisms on July 12, 2023 (KCCM13367P).

The *Bifidobacterium bifidum* P61 of the present disclosure is a Gram-positive bacterium, and the cell morphology is rod-shaped. More specifically, the physiological characteristics of *Bifidobacterium bifidum* P61 may be analyzed according to methods commonly used in the art.

Specifically, the physiological characteristics of *Bifidobacterium bifidum* P61 were analyzed by API kit test using carbon sources, and the results are shown in Table 1 below.

**[Table 1]**

| **Substrate** | **Reaction** |
|---|---|
| L-tryptophane | - |
| Urea | - |
| D-glucose | + |
| D-mannitol | - |
| D-lactose | + |
| D-saccharose | + |
| D-maltose | + |
| Salicin | - |
| D-xylose | + |
| L-arabinose | - |
| Gelatin | - |
| Esculin ferric citrate | + |
| Glycerol | - |
| D-cellobiose | - |
| D-mannose | + |
| D-melezitose | - |
| D-raffinose | + |
| D-sorbitol | - |
| L-rhamnose | - |
| D-trehalose | - |
| Catalase | - |
| Spore | - |
| Gram stain | + |
| Morphology(coccus) | - (Rod-shaped bacterium) |

The present disclosure provides *Lactobacillus paracasei* P62 (depositary institution: Korean Culture Center of Microorganisms, date of deposit: 2023.07.12, accession number: KCCM13368P).

The *Lactobacillus paracasei* P62 of the present disclosure is characterized in that it is a *lactic* acid bacterium isolated and identified from the intestinal microorganisms of a healthy person.

The 16S rDNA sequence for the identification and classification of *Lactobacillus paracasei* P62 of the present disclosure is the same as SEQ ID NO: 2 attached to this specification. Therefore, the *Lactobacillus paracasei P62* of the present disclosure may include 16S rDNA of SEQ ID NO: 2 as follows:

16S rDNA of Lactobacillus paracasei P62 (SEQ ID NO: 2):

ATGGCTCAGGATGAACGCTGGCGGCGTGCCTAATACATGCAAGTCGAACGAGTTCT
CGTTGATGATCGGTGCTTGCACCGAGATTCAACATGGAACGAGTGGCGGACGGGT
GAGTAACACGTGGGTAACCTGCCCTTAAGTGGGGGATAACATTTGGAAACAGATGC
TAATACCGCATAGATCCAAGAACCGCATGGTTCTTGGCTGAAAGATGGCGTAAGCTA
TCGCTTTTGGATGGACCCGCGGCGTATTAGCTAGTTGGTGAGGTAATGGCTCACCA
AGGCGATGATACGTAGCCGAACTGAGAGGTTGATGGCCACATTGGGACTGAGACA
CGGCCCAAACTCCTACGGGAGGCAGCAGTAGGGAATCTTCCACAATGGACGCAAG
TCTGATGGAGCAACGCCGCGTGAGTGAAGAAGGCTTTCGGGTCGTAAAACTCTGT
TGTTGGAGAAGAATGGTCGGCAGAGTAACTGTTGTCGGCGTGACGGTATCCAACCA
GAAAGCCACGGCTAACTACGTGCCAGCAGCCGCGGTAATACGTAGGTGGCAAGCG
TTATCCGGATTTATTGGGCGTAAAGCGAGCGCAGGCGGTTTTTTAAGTCTGATGTGA
AAGCCCTCGGCTTAACCGAGGAAGCGCATCGGAAACTGGGAAACTTGAGTGCAGA
AGAGGACAGTGGAACTCCATGTGTAGCGGTGAAATGCGTAGATATATGGAAGAACA
CCAGTGGCGAAGGCGGCTGTCTGGTCTGTAACTGACGCTGAGGCTCGAAAGCATG

As an analysis of the 16S rDNA base sequence of SEQ ID NO: 2, it showed 99% homology (homology with *Lactobacillus paracasei* R094 strain) and 98.6% homology (homology with *Lactobacillus paracasei* subsp. *paracasei* JCM8130 strain) with known *Lactobacillus paracasei* strains, and whole genome analysis also showed 98.7% homology, showing the highest molecular phylogenetic relationship with *Lactobacillus paracasei.* Therefore, the lactic acid bacterium was identified as *Lactobacillus paracasei* and named *Lactobacillus paracasei P62,* and was deposited with the Korean Culture Center of Microorganisms on July 12, 2023 (KCCM13368P).

The *Lactobacillus paracasei* P62 of the present disclosure is a Gram-positive bacterium, and the cell morphology is rod-shaped. More specifically, the physiological characteristics of *Lactobacillus paracasei* P62 may be analyzed according to conventional methods in the art.

Specifically, the physiological characteristics of *Lactobacillus paracasei P62* were analyzed by API kit test using a carbon source, and the results are shown in Table 2 below.

**[Table 2]**

| **Substrate** | **Reaction** | **Substrate** | **Reaction** |
|---|---|---|---|
| control | - | Esculin | + |
| Glycerol | - | Salicin | + |
| Erythritol | - | Cellobiose | + |
| D-arabinose | - | Maltose | + |
| L-arabinose | - | Lactose | + |
| D-ribose | + | Melibiose | - |
| D-xylose | - | Sucrose | - |
| L-oylose | - | Trehalose | + |
| D-adonitol | - | Inulin | - |
| Methyl-β-D-xylopyranoside | - | Melezitose | - |
| D-galactose | + | Raffinose | - |
| D-glucose | + | Starch | - |
| D-fructose | + | Glycogen | - |
| D-mannose | + | Xylitol | - |
| L-sorbose | + | Gentiobiose | + |
| Rhamnose | - | D-turanose | + |
| Dulcitol | - | D-lyxose | - |
| Inositol | - | D-tagatase | + |
| Mannitol | + | D-fucose | - |
| Sorbitol | + | 44. L-fucose | - |
| α-methyl-D-mannoside | - | D-arabitol | - |
| α-methyl-D-glucoside | - | L-arabitol | - |
| N-acetyl-glucosamine | + | Gluconate | + |
| Amygdalin | - | 2-keto-gluconate | - |
| Arbutin | + | 5-keto-gluconate | - |

The *Bifidobacterium bifidum* P61 KCCM13367P, *Lactobacillus paracasei* P62 KCCM13368P, or a mixture thereof of the present disclosure may treat sarcopenia by: (i) inhibiting expression of myofiber-degrading proteins MuRF-1 and MAFbx/Atrogin-1 and inflammatory cytokines TNF-α and IL-6; (ii) increasing AKT signaling activation which is a myofiber synthesis protein; (iii) inhibiting blood corticosterone and endotoxin concentrations that induce sarcopenia; and (iv) promoting muscle strength and muscle formation.

It also exhibits therapeutic effects for cognitive impairment by: (i) inhibiting behaviors similar to cognitive impairment; (ii) increasing the expression of BDNF and IL-10; and (iii) reducing the expression of TNF-α, IL-6, and IL-1β and blood endotoxin concentrations. Furthermore, it has an effect of alleviating inflammation by reducing the expression of inflammatory markers (TNF-α, IL-6, IL-1β, and MPO) and increasing the expression of anti-inflammatory markers (IL-10).

The present disclosure provides a pharmaceutical composition comprising *Bifidobacterium bifidum* P61 KCCM13367P, *Lactobacillus paracasei* P62 KCCM13368P, or a mixture thereof.

The present disclosure provides a pharmaceutical composition comprising *Bifidobacterium bifidum* P61 KCCM13367P, *Lactobacillus paracasei* P62 KCCM13368P, or a mixture thereof; and a pharmaceutically acceptable carrier.

The present disclosure provides a pharmaceutical composition for preventing or treating sarcopenia, cognitive impairment and inflammatory diseases comprising *Bifidobacterium bifidum* P61 KCCM13367P, *Lactobacillus paracasei* P62 KCCM13368P, or a mixture thereof.

As a specific embodiment for achieving the above object, the present disclosure provides a pharmaceutical composition for preventing or treating *sarcopenia* comprising *Bifidobacterium bifidum* P61 KCCM13367P, *Lactobacillus paracasei* P62 KCCM13368P, or a mixture thereof.

In the present disclosure, "sarcopenia" means a disease in which muscle mass decreases, and muscle volume and muscle strength gradually decline. In particular, it refers to a general term for diseases in which the muscles of the limbs gradually atrophy in an almost bilaterally symmetrical manner, which may accompany cancer, aging, kidney disease, hereditary diseases, and the onset of various chronic diseases. The sarcopenia may be at least one selected from the group consisting of, for example, muscular atrophy, disuse atrophy, spinal muscular amyotrophy, dystrophy, muscle rigidity, muscular hypotonia, muscle weakness, muscular dystrophy, amyotrophic lateral sclerosis, spinobulbar muscular atrophy, and myasthenia gravis, but is not limited thereto.

According to one embodiment of the present disclosure, it was confirmed that *Bifidobacterium bifidum* P61 KCCM13367P, *Lactobacillus paracasei* P62 KCCM13368P, or a mixture thereof exhibits an effect of alleviating sarcopenia by promoting muscle formation through effects such as: (i) inhibiting the expression of myofiber-degrading proteins MuRF-1 and MAFbx/Atrogin-1 and inflammatory cytokines TNF-α and IL-6; (ii) increasing muscle strength, muscle mass, and AKT signaling activation which is a myofiber synthesis protein; (iii) increasing the expression of MyHC, MyoG, PGC1-α, and SIRT-1; and (iv) reducing blood corticosterone and blood endotoxin.

As a specific embodiment for achieving the above object, the present disclosure provides a pharmaceutical composition for preventing or treating cognitive impairment comprising *Bifidobacterium bifidum* P61 KCCM13367P, *Lactobacillus paracasei* P62 KCCM13368P, or a mixture thereof.

In the present disclosure, "cognitive impairment" refers to a disorder showing cognitive impairment and behavioral changes, and refers to diseases arising from functional decline in memory, spatial perception, judgment, executive function, language ability, etc. The cognitive impairment may be any one or more selected from the group consisting of, for example, Alzheimer's disease, Huntington's disease, vascular dementia, Pick's disease, Parkinson's disease, Creutzfeldt-Jakob disease, and dementia, but is not limited thereto. In addition, the cognitive impairment encompasses memory impairment, dementia symptoms, etc. arising from the above symptoms.

According to one embodiment of the present disclosure, it has been confirmed that *Bifidobacterium bifidum* P61 KCCM13367P, *Lactobacillus paracasei* P62 KCCM13368P, or a mixture thereof exhibits an effect of ameliorating cognitive impairment by (i) inhibiting cognitive impairment-like behavior, (ii) increasing the expression of BDNF and IL-10, and (iii) reducing the expression of TNF-α, IL-6 and IL-1β and blood endotoxin concentration.

As a specific embodiment for achieving the above object, the present disclosure provides a pharmaceutical composition for preventing or treating inflammatory diseases comprising *Bifidobacterium bifidum* P61 KCCM13367P, *Lactobacillus paracasei* P62 KCCM13368P, or a mixture thereof.

In the present disclosure, "inflammatory disease" refers to a general term for diseases having inflammation as a main lesion, wherein the inflammatory disease may be any one or more selected from the group consisting of, for example, inflammatory bowel disease (IBD), arthritis, gout, hepatitis, obesity, gastritis, nephritis, diabetes, tuberculosis, bronchitis, pleurisy, peritonitis, spondylitis, pancreatitis, urethritis, cystitis, vaginitis, atherosclerosis, sepsis, and periodontitis, but is not limited thereto.

The "inflammatory bowel disease (IBD)" refers to a class of inflammatory conditions of the colon and gastrointestinal tract. Representative examples of inflammatory bowel disease include ulcerative colitis (UC) and Crohn's disease. The main difference between UC and Crohn's disease is the location and characteristics of the inflammatory changes. Crohn's disease may affect any part of the gastrointestinal tract from the mouth to the anus, whereas UC is limited in location of inflammation to the colon and rectum. Due to the specificity of presentation, a definitive diagnosis of Crohn's disease or ulcerative colitis cannot be made. In such cases, a diagnosis of indeterminate colitis may be made. Other forms of inflammatory bowel disease include, but are not limited to, collagenous colitis, lymphocytic colitis, ischemic colitis, diversion colitis, Behçet's disease, and indeterminate colitis.

That is, in the present disclosure, the inflammatory bowel disease may be any one or more selected from the group consisting of ulcerative colitis, Crohn's disease, collagenous colitis, lymphocytic colitis, ischemic colitis, diversion colitis, Behçet's disease, and indeterminate colitis.

According to one embodiment of the present disclosure, it was confirmed that *Bifidobacterium bifidum* P61 KCCM13367P, *Lactobacillus paracasei* P62 KCCM13368P, or a mixture thereof decreases the expression of inflammatory markers (TNF-α, IL-6, IL-1β, and MPO) and increases the expression of anti-inflammatory markers (IL-10), thereby exhibiting a therapeutic effect against inflammation.

In the present disclosure, "Muscle RING-finger protein-1 (MuRF-1)" refers to a signaling agent that degrades muscle proteins, and it is generally known that when MuRF-1 is activated, muscle atrophy proceeds.

In the present disclosure, "Atrogin-1" is a muscle-specific F-box protein that is involved in the process of degrading muscle protein, and because it is induced in the initial process of muscle atrophy, the increase precedes the decrease in muscle mass.

In the present disclosure, "tumor necrosis factor-α (TNF-α)" is a cytokine that is produced by macrophages in response to various immune stimuli, including endotoxins which are bacterial toxins, and plays an important role in the immune system. The abnormal regulation of the TNF-α is known to occur in various diseases, such as Alzheimer's disease, cancer, depression, and inflammatory bowel disease (IBD).

In the present disclosure, "interleukin 6 (IL-6)" is an interleukin that may act as both a pro-inflammatory cytokine and an anti-inflammatory myokine and plays the role of a differentiation factor involved in the activation of B lymphocytes and affects the function of various cells.

In the present disclosure, "FOXO3a" means one of the Forkhead box O family genes (FOXO1, FOXO3a, FOXO4, FOXO6) known as longevity genes, which regulates the expression of genes involved in cell death, cell cycle arrest, oxidative stress resistance, and autophagy.

In the present disclosure, "nuclear factor-kappa B (NF-κB)" means a protein complex involved in the transcription of DNA, the production of cytokines and the survival of cells, and is involved in the regulation of inflammatory response, the regulation of immune system, cell death, cell proliferation, the differentiation of epithelial cells, etc.

In the present disclosure, "AKT (protein kinase B, PKB)" is a general term for a set of three serine/threonine-specific protein kinases, which play an important role in cell cycle progression, smooth muscle cell differentiation, promotion of glucose uptake, angiogenesis, inhibition of apoptosis, and promotion of cell growth.

In the present disclosure, "mammalian target of rapamycin (mTOR)" is a serine-threonine kinase mammalian target of rapamycin, consisting of two separate protein complexes, mTOR complex 1 and mTOR complex 2, which regulate different cellular processes. The mTOR may function as a serine/threonine protein kinase that regulates cell growth, cell proliferation, cellular motility, cell survival, protein synthesis, autophagy, and transcription.

In the present disclosure, "myosin heavy chain (MyHC)" is a main contractile protein of sarcomeric filaments, which is an important component in maintaining muscle contraction.

In the present disclosure, "MyoG" means a gene encoding myogenin, which is involved in skeletal muscle development or regulation of muscle formation and repair.

In the present disclosure, "PGC1-α" is a key transcription factor activator that induces mitochondrial biogenesis in cells, and is known to regulate the composition and function of individual mitochondria.

In the present disclosure, "SIRT-1" is a gene encoding Sirtuin1, wherein the Sirtuin1 is a protein that functions in cellular responses to inflammation, metabolic and oxidative stress factors.

In the present disclosure, "brain-derived neurotrophic factor (BDNF)" is a brain-derived neurotrophic factor that acts on specific neurons in the central nervous system and the peripheral nervous system to help the survival of existing neurons and promote the growth and differentiation of new neurons and synapses.

In the present disclosure, "IL-10" means a potent immunosuppressive Th-2 cell cytokine produced by lymphocytes that functions to inhibit macrophage/monocyte and T lymphocyte replication and the secretion of inflammatory cytokines.

In the present disclosure, "IL-1β" is also referred to as leukocytic pyrogen, leukocytic endogenous mediator, mononuclear cell factor, or lymphocyte activating factor, and serves as a key mediator of inflammatory response.

In the present disclosure, "myeloperoxidase (MPO)" is a dimeric enzyme found mainly in azurophilic granules of neutrophils and lysosomes of monocytes, which promotes the formation of peroxides to primarily increase the microbicidal activity of innate immune response.

In the present disclosure, "corticosterone" is one of the adrenal cortical hormones and a type of representative glucocorticoid. The corticosterone is synthesized from pregnenolone or progesterone in the body and excreted via the kidney. The corticosterone is known to defend the body against stress, promote protein degradation, promote the synthesis of carbohydrates, inhibit inflammation, and exhibit an antagonistic effect on insulin.

In the present disclosure, "endotoxin" is a component of the outer membrane of Gram-negative bacteria known to react with the body's immune cells to induce an inflammatory reaction, and is also chemically referred to as a lipopolysaccharide (LPS).

The above results show that *Bifidobacterium bifidum* P61 KCCM13367P, *Lactobacillus paracasei* P62 KCCM13368P, or a mixture thereof have outstanding efficacy in preventing, treating or ameliorating sarcopenia, cognitive impairment and inflammatory diseases.

In addition, the pharmaceutical composition may have an excellent effect on the prevention, treatment or amelioration of sarcopenia, cognitive impairment and inflammatory diseases by changing the composition of the intestinal microbiome.

The form of the strain according to the present disclosure may be used in various forms, such as the form of a live cell, a dead cell, a culture, a lysate, or an extract. The strain according to the present disclosure, even if it has any form under conditions including the strain, shows results of an equivalent level or higher in terms of the above-mentioned effects (especially considering live cells).

The live cell means a living bacterium as it is, while the dead cell means that effective components have been separated and extracted by methods such as heat drying, pressurization, drug treatment, etc., after culturing viable bacteria under certain conditions.

The culture means a product obtained by cultivating lactic acid bacteria in a known liquid medium or solid medium, and is a concept including the strain according to the present disclosure. The product may include lactic acid bacteria. The medium may be selected from known liquid media or solid media, and may be, for example, MRS liquid medium, GAM liquid medium, MRS agar medium, GAM agar medium, BL agar medium, but is not limited thereto.

The lysate means having a crushed form by separating and processing live cells, dead cells, or cultures thereof through mechanical or chemical methods. For example, crushed forms may be prepared through bead mills, presses, sonicators or microfluidizers, enzymatic treatment, etc.

The extract means one obtained by extracting live cells, dead cells and/or lysates using commonly known extraction methods (known extraction solvents (for example, water, C1 to C4 alcohols (methanol, ethanol, etc.))).

In the present disclosure, it has been confirmed that a synergistic effect is exhibited in a mixture in which strains are mixed, and the present disclosure includes the use of any mixture thereof.

That is, by mixing *Bifidobacterium bifidum* P61 KCCM13367P and *Lactobacillus paracasei* P62 KCCM13368P according to the present disclosure, an excellent synergistic effect may be exhibited.

A mixture of *Bifidobacterium bifidum* P61 KCCM13367P and *Lactobacillus paracasei* P62 KCCM13368P may, for example, be included in a ratio of 10:1 to 1:10 based on the number of *colony* units (CFU), preferably in a ratio of 1:6 to 1:1, more specifically in a ratio of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9 or 1:10, and more preferably in a ratio of 1:4.

According to one embodiment of the present disclosure, the mixture of the strains exhibits an excellent synergistic effect against sarcopenia, cognitive impairment and inflammatory diseases (particularly, inflammatory bowel disease).

In particular, in terms of normalization of the intestinal microbiota, the mixture of the strains may exhibit a superior effect in terms of maintaining intestinal health compared to single strains.

The pharmaceutical composition according to the present disclosure may be manufactured into a pharmaceutical formulation using methods well known in the art to provide rapid, sustained, or delayed release of the active ingredient after administration to a mammal. In the manufacture of the formulation, the pharmaceutical composition according to the present disclosure may additionally include a pharmaceutically acceptable carrier within the range that does not inhibit the activity of the compound of the present disclosure.

The pharmaceutically acceptable carriers include, but are not limited to, those commonly used, such as lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinyl pyrrolidone, water, methylhydroxybenzoate, propylhydroxybenzoate, talc, magnesium stearate, and mineral oil. In addition, the pharmaceutical composition of the present disclosure may include diluents or excipients such as fillers, bulking agents, binders, wetting agents, disintegrants, surfactants, and other pharmaceutically acceptable additives.

Administration of the pharmaceutical composition according to the present disclosure may be carried out in a pharmaceutically effective amount. Here, "pharmaceutically effective amount" means an amount sufficient to prevent or treat a disease with a reasonable benefit/risk ratio applicable to medical treatment. The level of the effective amount may be variously selected by those skilled in the art depending on factors such as the formulation method, the condition and body weight of the patient, the patient's sex, age, severity of the disease, drug form, route and duration of administration, excretion rate, response sensitivity, etc. The effective amount may vary depending on the route of treatment, the use of excipients, and the possibility of use with other drugs as recognized by those skilled in the art. However, for desirable effects, for oral administration, the composition of the present disclosure may be administered to an adult generally at 0.0001 to 100 mg/kg of body weight per day, preferably 0.001 to 100 mg/kg of body weight per day, but the dosage does not limit the scope of the present disclosure in any respect.

The pharmaceutical composition according to the present disclosure may be administered to mammals such as mice, livestock, and humans through various routes.

Specifically, the pharmaceutical composition according to the present disclosure may be administered orally or parenterally (for example, topical application or intravenous, subcutaneous, intraperitoneal injection), but oral administration is preferred. Solid preparations for oral administration may include powders, granules, tablets, capsules, soft capsules, pills, etc. Liquid preparations for oral use include suspensions, solutions for internal use, emulsions, syrups, aerosols, etc., which may include various excipients such as wetting agents, sweeteners, flavoring agents, preservatives, etc., in addition to commonly used simple diluents such as water and liquid paraffin. Preparations for parenteral administration may be formulated and used in the form of sterile aqueous solutions, solutions, non-aqueous solvents, suspensions, emulsions, eye drops, ophthalmic ointments, syrups, suppositories, aerosols, and other external preparations and sterile injectable preparations, each sterilized according to conventional methods, and preferably pharmaceutical compositions of creams, gels, patches, sprays, ointments, liniments, lotions, liniments, ophthalmic ointments, eye drops, pastes, or cataplasms may be prepared and used, but are not limited thereto. Preparations for topical administration may be anhydrous or aqueous depending on clinical prescription. Non-aqueous solvents and suspensions may include propylene glycol, polyethylene glycol, vegetable oils such as olive oil, injectable esters such as ethyl oleate, etc. Bases for suppositories may include witepsol, macrogol, tween 61, cacao butter, laurin fat, glycerogelatin, etc.

The pharmaceutical composition according to the present disclosure may be administered alone as an individual therapeutic agent or in combination with other therapeutic agents, and may be administered sequentially or simultaneously with conventional therapeutic agents. Furthermore, the pharmaceutical composition of the present disclosure may be administered in single or multiple doses. Taking all of the above factors into consideration, it is important to administer an amount that may obtain maximum effect with a minimum amount without side effects, and this may be easily determined by those skilled in the art.

As used herein, the term "subject" includes an animal or human whose symptoms may be ameliorated by administration of a pharmaceutical composition according to the present disclosure. By administering a pharmaceutical composition according to the present disclosure to a subject, sarcopenia, cognitive impairment, or inflammatory diseases may be effectively prevented and treated.

The term "administration" in the present disclosure means introducing a predetermined substance to a human or animal by any appropriate method, and the administration route of the therapeutic composition according to the present disclosure may be administered orally or parenterally through any general route as long as it may reach the target tissue. Furthermore, the therapeutic composition according to the present disclosure may be administered by any device by which the active ingredient may move to target cells.

The preferred dosage of the pharmaceutical composition according to the present disclosure varies depending on the patient's condition and body weight, the degree of disease, drug form, administration route and duration, and may be appropriately selected by those skilled in the art.

The present disclosure provides *Bifidobacterium bifidum* P61 KCCM13367P, *Lactobacillus paracasei* P62 KCCM13368P, or a mixture thereof for use in the prevention or treatment of one or more diseases selected from the group consisting of sarcopenia, cognitive impairment and inflammatory diseases.

The present disclosure provides *Bifidobacterium bifidum* P61 KCCM13367P, *Lactobacillus paracasei* P62 KCCM13368P, or a mixture thereof for use in the prevention or treatment of *sarcopenia.*

The present disclosure provides *Bifidobacterium bifidum* P61 KCCM13367P, *Lactobacillus paracasei* P62 KCCM13368P, or a mixture thereof for use in the prevention or treatment of cognitive impairment.

The present disclosure provides *Bifidobacterium bifidum* P61 KCCM13367P, *Lactobacillus paracasei* P62 KCCM13368P, or a mixture thereof for use in the prevention or treatment of inflammatory diseases.

The present disclosure provides the use of *Bifidobacterium bifidum* P61 KCCM13367P, *Lactobacillus paracasei* P62 KCCM13368P, or a mixture thereof in the manufacture of a medicament for the treatment of one or more diseases selected from the group consisting of sarcopenia, cognitive impairment and inflammatory diseases.

The present disclosure provides the use of *Bifidobacterium bifidum* P61 KCCM13367P, *Lactobacillus paracasei* P62 KCCM13368P, or a mixture thereof in the manufacture of a medicament for the treatment of *sarcopenia.*

The present disclosure provides the use of *Bifidobacterium bifidum* P61 KCCM13367P, *Lactobacillus paracasei* P62 KCCM13368P, or a mixture thereof in the manufacture of a medicament for the treatment of cognitive impairment.

The present disclosure provides the use of *Bifidobacterium bifidum* P61 KCCM13367P, *Lactobacillus paracasei* P62 KCCM13368P, or a mixture thereof in the manufacture of a medicament for the treatment of inflammatory diseases.

The present disclosure provides a method for preventing or treating sarcopenia, cognitive impairment, and inflammatory diseases, comprising a step of administering *Bifidobacterium bifidum* P61 KCCM13367P, *Lactobacillus paracasei* P62 KCCM13368P, or a mixture thereof to a subject in need thereof.

The present disclosure provides a composition comprising *Bifidobacterium bifidum* P61 KCCM13367P, *Lactobacillus paracasei* P62 KCCM13368P, or a mixture thereof.

The present disclosure provides a food composition for preventing or ameliorating sarcopenia, cognitive impairment, and inflammatory diseases, comprising *Bifidobacterium bifidum* P61 KCCM13367P, *Lactobacillus paracasei* P62 KCCM13368P, or a mixture thereof.

The terms *"Bifidobacterium bifidum* P61", "Lactobacillus *paracasei* P62", "sarcopenia", "cognitive impairment" and "inflammatory diseases" are the same as described above.

Specifically, the lactic acid bacteria included in the food composition according to the present disclosure may be live cells thereof, dead cells thereof, a culture thereof, lysates thereof, or an extract thereof, but any form of lactic acid bacteria that may achieve the effect of preventing or ameliorating sarcopenia, cognitive impairment, or inflammatory disease disorders may be used without limitation.

There is no particular limitation on the type of the food. The foods to which the lactic acid bacteria may be added include sausages, meat, bread, chocolates, snacks, candies, confectionery, instant noodles, pizza, other noodles, gums, dairy products including ice cream, various soups, beverages, tea, drinks, alcoholic beverages, and vitamin complexes. When formulated as a beverage, the liquid components added in addition to the novel lactic acid bacteria are not limited thereto, but may contain various flavoring agents or natural carbohydrates as additional components, as in conventional beverages. The aforementioned natural carbohydrates may be monosaccharides (e.g., glucose, fructose, etc.), disaccharides (e.g., maltose, sucrose, etc.) and polysaccharides (e.g., conventional sugars such as dextrin, cyclodextrin, etc.), and sugar alcohols such as xylitol, sorbitol, erythritol, etc.

The type of the food may specifically be a health functional food. The health functional food may contain various nutrients, vitamins, minerals (electrolytes), flavoring agents such as synthetic flavoring agents and natural flavoring agents, coloring agents and enhancers (cheese, chocolate, etc.), pectic acid and salts thereof, organic acids, protective colloidal thickening agents, pH adjusters, stabilizers, preservatives, glycerin, alcohol, carbonating agents used in carbonated beverages, etc. These components may be used alone or in combination, and the proportion of these additives is generally selected in the range of 0.001 to 50 parts by weight per total weight of the composition.

The health functional food is a food that emphasizes the bioregulatory function of food and is a food to which added value has been imparted to act and express for a specific purpose using physical, biochemical, and biotechnological methods. The components of such health functional food are designed and processed to sufficiently exert bioregulatory functions related to biological defense and regulation of body rhythm, prevention and recovery of diseases on the living body, and may contain food auxiliary additives, sweeteners, or functional raw materials that are acceptable as food.

When the *Bifidobacterium bifidum* P61 KCCM13367P and/or *Lactobacillus paracasei* P62 KCCM13368P of the present disclosure are used as a health functional food (or health functional beverage additive), the *Bifidobacterium bifidum* P61 KCCM13367P and/or *Lactobacillus paracasei* P62 KCCM13368P may be added as such or used together with other foods or food components, and may be appropriately used according to conventional methods. The mixing amount of the *Bifidobacterium bifidum* P61 KCCM13367P and/or *Lactobacillus paracasei* P62 KCCM13368P may be appropriately determined according to the purpose of use thereof (prevention, health or amelioration, therapeutic treatment).

The novel lactic acid bacteria of the present disclosure and a mixture thereof modulate intestinal microbial composition, thereby inhibiting the expression of sarcopenia markers (MuRF-1, MAFbx/Atrogin-1, etc.), increasing muscle strength and muscle mass, and promoting muscle formation to alleviate sarcopenia. Furthermore, they inhibit behaviors similar to cognitive impairment and regulate the expression of factors associated with the factors(BDNF, IL-10, etc.), thereby exhibiting therapeutic effects against cognitive impairment. In addition, they have an effect of alleviating inflammation by decreasing the expression of inflammatory markers (TNF-α, IL-6, IL-1β, and MPO) and increasing the expression of anti-inflammatory markers (IL-10).

### Brief Description of Drawings

FIG. 1 is a diagram confirming the changes in the expression of sarcopenia-related factors (MuRF-1, MAFbx/Atrogin-1, TNF-α, and IL-6) and the activation of NF-κB (p65) for each mouse model (Yg(NC), Vh, Lp, Bb, LB, and Cr). (The data in FIG. 1 are represented as mean ± SD (n = 4). #p<0.05 vs NC. *p<0.05 vs. dexamethasone or LPS alone-treated group.)
FIG. 2 is a diagram showing changes in the number of NF-κB+CD11c+ cells in each mouse model (Yg, Vh, Lp, Bb, LB, and Cr), as observed through immunofluorescence staining.
FIG. 3 is a diagram showing changes in the size of gastrocnemius muscle cells (H&E staining), the number of MyHC-positive cells (immunofluorescence staining), and the cross-sectional area (CSA) of the muscle in each mouse model (Yg, Vh, Lp, Bb, LB, and Cr).
FIG. 4 is a drawing showing changes in the number of NF-κB+Iba1+ cells and BDNF+NeuN+ cells in each mouse model (Yg, Vh, Lp, Bb, LB, and Cr), as confirmed through immunofluorescence staining.

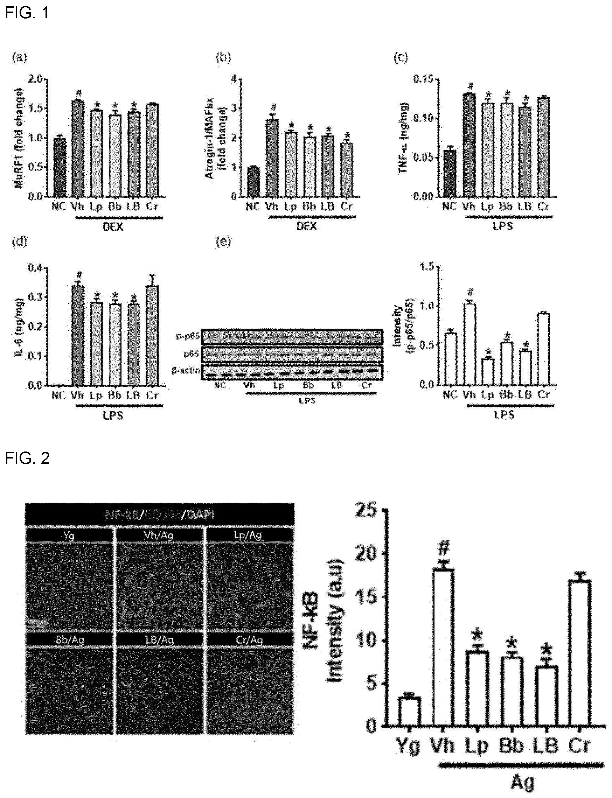

(Data in FIGS. 2 to 4 are represented as mean ± SD (n = 7). #p<0.05 vs. Yg. *p<0.05 vs. Vh/Ag.)

### Description of Embodiments

Hereinafter, preferred embodiments are presented to aid understanding of the present disclosure. However, the following embodiments are provided only for easier understanding of the present disclosure, and the content of the present disclosure is not limited thereby.

### Experimental Example 1. Isolation and Culture of Lactic Acid Bacteria

*Bifidobacterium bifidum* P61 and *Lactobacillus paracasei* P62, isolated from human intestinal microflora, were cultured in MRS medium (BD, Sparks, MD) at room temperature, then isolated and collected. The collected cells were freeze-dried, then washed twice with saline and suspended for in vitro experiments, and suspended in 1% maltose solution for in vivo experiments.

### Experimental Example 2. C2C12 Cell Culture

C2C12 cells (Korean Type Culture Collection) were cultured in DMEM medium containing 10% fetal bovine serum (FBS), 1% antibiotic-antifungal solution, and 3.7g/L NaHCO₃ at 37°C in a 5% CO₂/95% humidified air incubator. Confluent myoblasts (80%) were differentiated in DMEM medium containing 2% horse serum for 4 days.

### Experimental Example 3. Preparation of Mouse Model

For the preparation of the mouse model, aging C57BL/6 mice (male, 18 months old) and young C57BL/6 mice (male, 3 months old) were purchased and used from Orientbio Co., Ltd. Furthermore, all animal experiments carried out for the present disclosure were approved in advance by the Kyung Hee University Institutional Animal Care and Use Committee (IACUC No. KHUASP(SE)-22-567) and were conducted ethically in accordance with the university guidelines for the care and use of laboratory animals.

The mice were divided into the following six groups, with each group including eight mice.
Yg(NC): a group in which young C57BL/6 mice were treated with vehicle.
Vh: a group in which aging C57BL/6 mice were treated with vehicle.
Bb: a group in which aging C57BL/6 mice treated with *Bifidobacterium bifidum* P61 (1×10⁹ CFU/mouse).
Lp: a group in which aging C57BL/6 mice treated with *Lactobacillus paracasei* P62 (1×10⁹ CFU/mouse).
LB: a group in which aging C57BL/6 mice treated with a 1:4 mixture of *Bifidobacterium bifidum* P61 and *Lactobacillus paracasei* P62.
Cr: Aging C57BL/6 mice treated with creatine (75 mg/kg).

At this time, the treatment of the preparations in each of the above groups was carried out orally through gavage once a day for 8 weeks (6 days per week).

### Experimental Example 4. Motor Ability Measurement and Cognitive Function Test Method

The motor ability of mice was measured 20 hours after the last treatment with the formulation in each group of Experimental Example 3. Specifically, the grip strength test was performed by placing the limbs of the mouse on a grid of a grip strength meter and measuring the grip strength just before the mouse fell from the bar. In addition, the treadmill test was performed by adapting the mice to a treadmill for one week and having them run at a speed of 23 m/min for 30 minutes to measure running time and distance.

The cognitive function test was performed through a Y-maze test, which used a Y-maze measuring device with three arms extending in the shape of the letter Y, each arm having a length of 25 cm, a height of 14 cm, a width of 5 cm, and positioned at the same angle.

### Experimental Example 5. ELISA and immunoblotting

Mouse gastrocnemius muscle, colon, brain tissue and cells were homogenized on ice using RIPA lysis buffer containing 1% phosphatase inhibitor cocktail and 1% protease inhibitor cocktail (RPP) and centrifuged at 4°C, 15,000 g for 15 minutes. Thereafter, the supernatant of the homogenate was transferred to a PVDF membrane after sodium dodecyl sulfate-polyacrylamide gel (SDS-PAGE) electrophoresis and immunoblotting was performed. Proteins were detected through antibodies, detected with HRP-conjugated secondary antibodies and visualized with an ECL detection kit. Meanwhile, cytokines were analyzed by transferring the supernatant of the homogenate to a 96-well plate using an ELISA kit (R&D System).

The antibodies used at this time were as follows: phospho-AKT (Ser473) (193H12, Cell Signaling, Danvers, MA), AKT (11E7) (4685, Cell Signaling), MAFbx (F-9) (sc-166806, Santa Cruz Biotechnology, Santa Cruz, CA), MuRF-1 (C-11) (sc-398608, Santa Cruz Biotechnology), phospho-mTOR (ser2448) (2971, Cell Signaling), mTOR(7C10) (2983, Cell Signaling), p-NF-κB-p65 (S536) (93H1, Cell Signaling), NF-κB-p65 (D14E12) (8242, Cell Signaling), MYH (B-5) (sc-376157, Santa Cruz Biotechnology), p16INK4A (E5F3Y, Cell signaling), p-FOXO3a (ser253, Cell signaling), FOXO3a (75D8, Cell signaling), β-actin (sc-47778, Santa Cruz Biotechnology), and PGC1α (ab191838, Abcam).

### Experimental Example 6. Real-time polymerase chain reaction (qPCR) analysis

Total RNA was purified from gastrocnemius muscle and C2C12 cells using an RNeasy mini kit (Qiagen) and an RNeasy Fibrous Tissue mini kit (Qiagen), respectively. 2 µg of the purified RNA was reverse transcribed using a PrimeScript cDNA synthesis kit (Takara), and PCR was performed using TB Green Premix Ex Taq II (Takara) and a Rotor-Gene Q 5plex Platform (Qiagen).

Meanwhile, total DNA was purified from gastrocnemius muscle and C2C12 cells using a Genomic DNeasy kit (Qiagen), and the mtDNA copy number was measured by qPCR.

The primers used at this time are as shown in Table 3.

**[Table 3]**

| **Gene** | **Primer sequence** | |
|---|---|---|
| | **Forward** | **Reverse** |
| **MuRF-1** | 5'- AGCCAAGACAATAGAGATGCCTACTTC- 3'(SEQ ID NO: 3) | 5'-GGCCTTGAACTCATAGAGATCCAAC- 3' (SEQ ID NO: 4) |
| **MAFbx/Atrogin1** | 5'-AAGGAAGATGAACGCTGTCA-3'(SEQ ID NO: 5) | 5'-ATTGCCTCCCAGATAAAGTATGT-3' (SEQ ID NO: 6) |
| **TNF-α** | 5'-GATTATGGCTCAGGGTCCAA-3' (SEQ ID NO: 7) | 5'-GCTCCAGTGAATTCGGAAAG-3' (SEQ ID NO: 8) |
| **IL-6** | 5'-TAGTCCTTCCTACCCCAATTTCC-3' (SEQ ID NO: 9) | 5'-TTGGTCCTTAGCCACTCCTTC-3' (SEQ ID NO: 10) |
| **MyHC** | 5'-ACAAGCTGCGGGTGAAGAGC-3' (SEQ ID NO: 11) | 5'-CAGGACAGTGACAAAGAACG-3' (SEQ ID NO: 12) |
| **MyHC-I** | 5'-GCAAGACGGTGACTGTGAAGGAG- 3'(SEQ ID NO: 13) | 5'-GGTTGACGGTGACGCAGAAGAG- 3'((SEQ ID NO: 14) |
| **MyHC-2A** | 5'-AAGCGAAGAGTAAGGCTGTC-3' (SEQ ID NO: 15) | 5'-GTGATTGCTTGCAAAGGAAC-3' (SEQ ID NO: 16) |
| **MyHC-2X** | 5'-CACCGTCTGGATGAGGCTGA-3' (SEQ ID NO: 17) | 5'-TGTTTGCGCAGACCCTTGATAG-3' (SEQ ID NO: 18) |
| **MyHC-2B** | 5'-GATTGACGTGGAGAGGTCTAAC-3' (SEQ ID NO: 19) | 5'-CCTGAGTTTCCTCGTACTTCTG-3' (SEQ ID NO: 20) |
| **MyoG** | 5'-GGCTGCCTAAAGTGGAGATCCT-3' (SEQ ID NO: 21) | 5'-AGGCCTGTAGGCGCTCAAT-3' (SEQ ID NO: 22) |
| **PGC1-α** | 5'-TGATGTGAATGACTTGGATACAGACA- 3'(SEQ ID NO: 23) | 5'-GCTCATTGTTGTACTGGTTGGATATG- 3'(SEQ ID NO: 24) |
| **SIRT1** | 5'-AGGGAACCTTTGCCTCATCTAC-3' (SEQ ID NO: 25) | 5'-GGTGGCAACTCTGATAAATGAAC- 3'(SEQ ID NO: 26) |
| **mtDNA** | 5'-TTTTATCTGCATCTGAGTTTAA-3' (SEQ ID NO: 27) | 5'-CCACTTCATCTTACCATTTAT-3' (SEQ ID NO: 28) |
| **β-actin** | 5'-CCATCCTGCGTCTGGACCTG-3' (SEQ ID NO: 29) | 5'-CTCGTCATACTCCTGCTTGC-3' (SEQ ID NO: 30) |

### Experimental Example 7. Hematoxylin & Eosin (H&E) Staining and Immunofluorescence Staining

After paraformaldehyde was injected into the heart of the mice, the gastrocnemius muscle of the hind limbs was sectioned into 5 µm slices and stained with hematoxylin & eosin (H&E).

In addition, hypothalamus, gastrocnemius muscle, and colon tissues were collected from the mice, sectioned, and incubated with primary antibodies (BDNF, NeuN, p65, NF-κB, Iba1, and/or CD11c) for 12 hours. Subsequently, the sections were treated with secondary antibodies conjugated with Alexa Fluor594 or Alexa Fluor 488 and observed through a confocal microscope.

### Embodiment 1. Alleviation of Sarcopenia by Bifidobacterium bifidum P61 and Lactobacillus paracasei P62

### (1) Confirmation of changes in expression of MuRF-1, MAFbx/Atrogin-1, TNF-α and IL-6, and activation of NF-κB

C2C12 cells (1x10⁵ cells/mL) of the mouse groups (Yg, Vh, Bb, Lp, LB and Cr) prepared in Experimental Example 3 were treated with 0.3 mM dexamethasone or 100 ng/mL LPS, and changes in the expression of myofiber-degrading proteins (MuRF-1, MAFbx/Atrogin-1), inflammatory cytokines (TNF-α and IL-6), and the activation of NF-κB were observed.

As a result, it was confirmed that *Bifidobacterium bifidum* P61 and/or *Lactobacillus paracasei* P62 inhibited the expression of MuRF-1 and MAFbx/Atrogin-1 induced by dexamethasone (FIGS. 1A and 1B). Additionally, it was confirmed that the expression of TNF-α and IL-6 and NF-κB activation induced by lipopolysaccharide (LPS) were significantly inhibited (FIGS. 1C to 1E).

That is, it was confirmed that the expression of sarcopenia-related factors and the activation of NF-κB were ameliorated by *Bifidobacterium bifidum* P61, *Lactobacillus paracasei* P62, or a mixture thereof, thereby alleviating *sarcopenia.*

### (2) Confirmation of muscle strength, muscle mass changes and AKT signal activation

An experiment was conducted to confirm muscle strength and muscle mass changes for the mouse groups (Yg, Vh, Bb, Lp, LB and Cr) prepared in Experimental Example 3, and during the experiment, no significant differences in food intake and weight gain of the aging mouse models were observed between the groups.

In order to verify the changes in muscle strength, treadmill tests and grip strength tests were conducted on each mouse group.

As a result of conducting the treadmill test, it was confirmed that treatment with *Bifidobacterium bifidum* P61 and/or *Lactobacillus paracasei* P62 increased the running time and distance of the aging mouse model (Tables 4A and 4B). Among these, the group (LB) treated with a 1:4 mixture of *Bifidobacterium bifidum* P61 and *Lactobacillus paracasei* P62 showed the most significant increase in running time and distance. In addition, in the grip strength test, it was confirmed that the grip strength intensity of the groups (Bb, Lp, and LB) treated with *Bifidobacterium bifidum* P61 and/or *Lactobacillus paracasei* P62 increased by approximately 1.15-fold, 1.14-fold, and 1.16-fold, respectively, compared to the Vh group (Table 4C).

Next, to confirm changes in muscle mass, weight changes of the gastrocnemius (GA), soleus (SOL), quadriceps (QD), extensor digitorum longus (EDL), and tibialis anterior (TA) in each group were observed. As a result, the weights of GA, SOL, QD, EDL, and TA in the aging mouse model were significantly increased by treatment with *Bifidobacterium bifidum* P61 and/or *Lactobacillus paracasei* P62 (Tables 4D to 4H), and accordingly, the total muscle weight also showed an increasing pattern (Table 4I).

The specific results of each of the above experiments are shown in Table 4 below.

**[Table 4]**

| | **Mean ± SD** | | | | | |
|---|---|---|---|---|---|---|
| | **Yg** | **Ag** | **Lp** | **Bb** | **LB** | **Cr** |
| **(a) Running distance (m)** | 640.1±28.6 | 104.9±71.4 | 335.8±155.7 | 320.1±118.9 | 500.7±1215 | 256.9±198.4 |
| **(b) Running time (s)** | 1768.3±77.6 | 325.9±175.8 | 950.5±421.9 | 907.4±320.8 | 1305.5±525.8 | 772.5±511.7 |
| **(c) Grip strength (N)** | 3.12±0.1 | 2.18±0.1 | 2.48±0.2 | 2.51±0.1 | 2.52±0.1 | 2.35±0.1 |
| **(d) GA weight (mg/g)** | 11.88±0.2 | 9.51±0.4 | 10.29±0.8 | 10.33±0.7 | 10.37±0.6 | 10.21±0.5 |
| **(e) SOL weight (mg/g)** | 0.7±0.0 | 0.53±0.0 | 0.63±0.0 | 0.64±0.0 | 0.68±0.0 | 0.67±0.0 |
| **(f) QD weight (mg/g)** | 15.2±0.8 | 11.66±0.5 | 12.56±0.5 | 12.97±02 | 13.02±0.6 | 12.81±0 7 |
| **(g) EDL weight (mg/g)** | 0.87±0.0 | 0.72±0.0 | 0.73±0.1 | 0.75±0.1 | 0.77±0.1 | 0.78±0.0 |
| **(h) TA weight (mg/g)** | 3.71±0.3 | 3.14±0.1 | 3.41±0.1 | 3.53±0.1 | 3.57±0.1 | 3.45±0.2 |
| **(i) Total weight (mg/g)** | 32.37±0,8 | 25.56±0.7 | 27.42±0.8 | 28.22±1 | 28.41±1 | 27.86±1.2 |

In addition, it was confirmed that AKT and mTOR activation related to protein synthesis also increased in the gastrocnemius muscle of the groups treated with *Bifidobacterium bifidum* P61 and/or *Lactobacillus paracasei* P62 (Bb, Lp and LB), and this is shown in Table 5 below.

**[Table 5]**

| | **Mean ± SD** | | | | | |
|---|---|---|---|---|---|---|
| | **Yg** | **Ag** | **Lp** | **Bb** | **LB** | **Cr** |
| **(a) Intensity (p-Akt/Akt)** | 0.4±0.0 | 0.1±0.0 | 0.5±0.0 | 0.6±0.0 | 1.1±0.2 | 0.7±0.0 |
| **(b) intensity (p-mTOR/mTOR)** | 0.8±0.3 | 0.4±0.0 | 0.7±0.0 | 1.1±0.1 | 0.7±0.0 | 0.3±0.2 |
| **(c) Intensity (p-FOXO3a/FOXO3a)** | 0.7±0.0 | 1.4±0.0 | 1.0±0.1 | 0.9±0.0 | 0.6±0.0 | 1.4±0.0 |
| **(d) Intensity (p-p65 NF-κB/p65 NF-κB)** | 0.4±0.1 | 1.5±0.1 | 0.5±0.0 | 0.4±0.0 | 0.4±0.0 | 1.4±0.0 |

That is, it was confirmed that *Bifidobacterium bifidum* P61, *Lactobacillus paracasei* P62, or a mixture thereof increased muscle strength, muscle mass, and AKT signaling activation, and decreased FOXO3a and NF-κB activation, thereby exhibiting a therapeutic effect for *sarcopenia.*

### (3) Confirmation of expression changes in myogenesis genes

qPCR, immunoblotting, H&E staining, and immunofluorescence staining were performed on the mouse groups (Yg, Vh, Bb, Lp, LB, and Cr) prepared in Experimental Example 3 to confirm expression changes in myogenesis genes due to administration of *Bifidobacterium bifidum* P61 and/or *Lactobacillus paracasei* P62.

Specifically, qPCR results showed that MuRF-1, MAFbx/Atrogin-1, TNF-α and IL-6 expression decreased in the groups treated with *Bifidobacterium bifidum* P61 and/or *Lactobacillus paracasei* P62 (Bb, Lp and LB). In contrast, MyHC and MyoG expression increased, and in particular, the expression of MyHC 2X, MyHC 2B, and MyHC showed a significant increase compared to the creatine-treated group (Cr). Furthermore, the level of mtDNA in the gastrocnemius muscle was restored to a level similar to that of young mice, and a significant increase in the expression levels of PGC1-α and SIRT-1, which are involved in mitochondrial gene expression, was observed.

The specific results for this are shown in Table 6 below.

**[Table 6]**

| | **Mean ± SO** | | | | | |
|---|---|---|---|---|---|---|
| | **Yg** | **Ag** | **Lp** | **Bb** | **LB** | **Cr** |
| **(a) MuRF1 (fold change)** | 1.0±0.2 | 1.4±0.1 | 1.3±0.2 | 1.1±0.1 | 1.1±0.1 | 1.3±0.2 |
| **(b)MAFbx/Atrogin-1 (fold change)** | 1.0±0.1 | 1.4±0.1 | 1.2±0.2 | 1.1±0.2 | 1.1±0.1 | 1.2±0.2 |
| **(c) TNF-α (fold change)** | 1.0±0.3 | 1.7±0.1 | 1.4±0.1 | 1.2±0.1 | 1.1±0.1 | 1.5±0.1 |
| **(d) IL-6 (fold change)** | 1.0±.0.1 | 2.0±0.2 | 1.4±0.2 | 1.4±0.1 | 1.4±0.1 | 1.7±0.2 |
| **(e) MyHC (fold change)** | 1.0±0.1 | 0.7±0.1 | 1.3±0.0 | 0.9±0.1 | 1.1±0.1 | 0.8±0.1 |
| **(f) MyHC 2A (fold change)** | 1.0±0.2 | 0.7±0.1 | 1.2±0.1 | 0.8±0.2 | 1.1±0.1 | 0.8±0.2 |
| **(g) MyHC 2X (fold change)** | 1.0±0.1 | 0.7±0.1 | 1.2±0.0 | 1.1±0.1 | 1.2±0.2 | 1.0±0.2 |
| **(h) MyHC 2B (fold change)** | 1.0±0.0 | 0.7±0.1 | 1.0±0.1 | 0.9±0.1 | 1.0±0.1 | 0.8±0.1 |
| **(i) MyoG (fold change)** | 1.0±0.1 | 0.7±0.1 | 0.9±0.2 | 0.8±0.1 | 0.9±0.0 | 0.9±0.1 |
| **(j) PGC-1α (fold change)** | 1.0±0.2 | 0.7±0.1 | 1.0±0.1 | 1.2±0.1 | 1.2±0.2 | 1.1±0.1 |
| **(k) SIRT1 (fold change)** | 1.0±0.1 | 0.7±0.2 | 1.4±0.1 | 1.5±0.1 | 1.5±0.2 | 1.6±0.2 |
| **(l) mtDNA (fold change)** | 1.0±0.1 | 0.8±0.1 | 0.9±0.1 | 0.9±0.1 | 1.0±0.1 | 0.9±0.0 |

According to ELISA, *Bifidobacterium bifidum* P61 and/or *Lactobacillus paracasei* P62 reduced the aging-induced increase in blood corticosterone as shown in Table 7 below.

**[Table 7]**

| | **Mean ± SD** | | | | | |
|---|---|---|---|---|---|---|
| | **Yg** | **Ag** | **Lp** | **Bb** | **LB** | **Cr** |
| **Corticosterone (ng/mL)** | 191.7±29.8 | 392.8±23.4 | 280.0±35.1 | 282.0±33.2 | 244.2+31.2 | 315.0±29.1 |

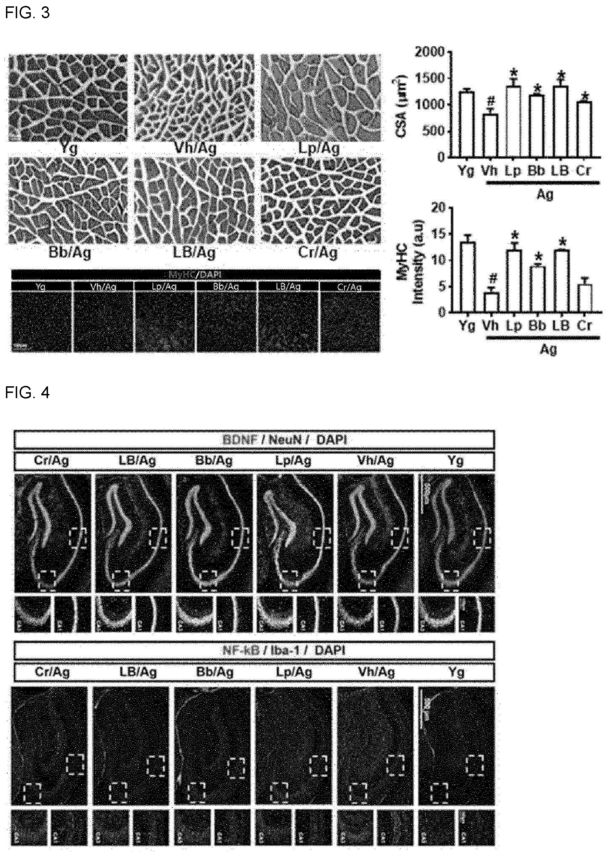

In addition, when muscle cells were observed after H&E staining or immunofluorescence staining, the number of NF-κB+CD11c+ cells decreased in the groups (Bb, Lp, and LB) treated with *Bifidobacterium bifidum* P61 and/or *Lactobacillus paracasei* P62 (FIG. 2), while the size of gastrocnemius muscle cells (H&E staining) and the number of MyHC-positive cells (immunofluorescence staining) increased, and the cross-sectional area (CSA) of the muscle was restored to a level similar to that of the young mouse model (Yg) (FIG. 3).

That is, it was confirmed that *sarcopenia* may be ameliorated by promoting muscle formation by *Bifidobacterium bifidum* P61, *Lactobacillus paracasei* P62, or a mixture thereof.

### Embodiment 2. Alleviation of cognitive impairment by Bifidobacterium bifidum P61 and Lactobacillus paracasei P62

A Y-maze test was performed on the mouse groups (Yg, Vh, Bb, Lp, LB and Cr) prepared in Experimental Example 3 to measure spontaneous alternation, and changes in expression of cognitive function-related factors were confirmed.

The aged mouse models (Bb, Lp and LB) treated with *Bifidobacterium bifidum* P61 and/or *Lactobacillus paracasei* P62 showed a tendency of increased spontaneous alternation in the Y-maze test, confirming that cognitive impairment-like behavior was inhibited (Table 8A). In addition, expression of BDNF and IL-10 was increased in the hippocampus of the aged mouse models, expression of TNF-α, IL-6 and IL-1β was inhibited (Tables 8B to 8H), and the number of NF-κB⁺Iba1⁺ cells and BDNF⁺NeuN⁺ cells was increased (FIG. 4).

The specific results for this are shown in Table 8 and FIG. 4 below.

**[Table 8]**

| | **Mean ± SD** | | | | | |
|---|---|---|---|---|---|---|
| | **Yg** | **Ag** | **Lp** | **Bb** | **LB** | **Cr** |
| **(a) Spon. alternation (%)** | 74.4±4.7 | 49.8±8.3 | 63.4±5.6 | 57.0+6.3 | 72.6±5.0 | 57.0±6.3 |
| **(b) BDNF (pg/mg)** | 342.4±13.4 | 301.2±17.6 | 330.3±23.6 | 328.7±11.7 | 328.3±11.8 | 313.8±22.9 |
| **(c) TNF-α (ng/mg)** | 0.22±0.01 | 0.28±0.01 | 0.26±0.01 | 0.25±0.01 | 0.25±0.01 | 0.27±0.02 |
| **(d) IL-6 (pg/mg)** | 46.3±2.6 | 59.7±2.1 | 53.7±1.9 | 53.6+0.8 | 51.9±2.2 | 55.3±5.7 |
| **(e) IL-1β (pg/mg)** | 12.0±1.2 | 17.8±1.5 | 15.8±0.7 | 15.6±0.7 | 15.5±1.3 | 16.5±1.6 |
| **(f) IL-10 (pg/mg)** | 46.7±3.6 | 41.1±4.3 | 46.0±1.1 | 46.5±3.3 | 48.4±2.2 | 43.8±2.3 |
| **(g) TNF-α/IL-10** | 4.6±0.3 | 6.9±0.9 | 5.6±0.3 | 5.5±0.5 | 5.3±0.4 | 6.1±0.6 |
| **(h) IL-6/IL-10** | 1.0±0.1 | 1.4±0.1 | 1.2±0.1 | 1.2±0.1 | 1.1±0.1 | 1.3±0.1 |

In addition, administration of *Bifidobacterium bifidum* P61 and/or *Lactobacillus paracasei* P62 reduced endotoxin that causes systemic inflammation and cognitive impairment. The specific results for this are shown in Table 9 below.

**[Table 9]**

| | **Mean ± SD** | | | | | |
|---|---|---|---|---|---|---|
| | **Yg** | **Ag** | **Lp** | **Bb** | **LB** | **Cr** |
| **Endotoxin (EU/mL)** | 442.9±25.7 | 502.9±40.1 | 429.5±28.6 | 439.2±20 | 436.7±24.6 | 482.9±24.3 |

That is, it was confirmed that a therapeutic effect against cognitive impairment was exhibited by administering *Bifidobacterium bifidum* P61, *Lactobacillus paracasei* P62, or a mixture thereof.

### Embodiment 3. Confirmation of inflammation alleviation by Bifidobacterium bifidum P61 and Lactobacillus paracasei P62

To evaluate the anti-inflammatory effect, qPCR and immunofluorescence staining were performed on the colon of mouse models (Yg, Vh, Bb, Lp, LB and Cr) prepared in Experimental Example 3.

As a result of qPCR, the groups treated with *Bifidobacterium bifidum* P61 and/or *Lactobacillus paracasei* P62 (Bb, Lp and LB) showed decreased expression of inflammatory markers TNF-α, IL-6, IL-1β and myeloperoxidase (MPO) and increased expression of anti-inflammatory marker IL-10.

The specific results for this are shown in Table 10 below.

**[Table 10]**

| | **Mean ± SD** | | | | | |
|---|---|---|---|---|---|---|
| | **Yg** | **Ag** | **Lp** | **Bb** | **LB** | **Cr** |
| **(a) TNF-α (pg/mg)** | 29.1±4.8 | 43.4±6.6 | 32.4±4.9 | 33.7±2.4 | 29.5±5.0 | 36.6±6.4 |
| **(b) IL-6 (pg/mg)** | 10.1±1.3 | 14.6±2.5 | 11.5±1.5 | 11.9±1.6 | 10.9±2.2 | 12.2±2.2 |
| **(c) IL-10 (pg/mg)** | 52.7±3.2 | 35.4±7.8 | 48.7±4.8 | 49.6±3.8 | 59.1±6.7 | 36.7±4.9 |
| **(d) IL-1β (pg/mg)** | 23.6±3.3 | 30.3±1.5 | 25.1±3.8 | 25.1±2.0 | 22.9±4.7 | 28.3±4.4 |
| **(e) MPO (ng/mg)** | 3.1±0.6 | 5.4±0.9 | 3.8±0.6 | 3.7±0.6 | 3.7±0.4 | 5.1 ±0.9 |
| **(f) TNF-α /IL-10** | 0.6±0.1 | 1.2±0.2 | 0.7±0.1 | 0.7±0.0 | 0.5±0.1 | 1.0±0.2 |
| **(g) IL-6/IL-10** | 0.2±0.0 | 0.4±0:1 | 0.2±0.0 | 0.2±0.0 | 0.2±0.1 | 0.3±0.1 |
| **(h) NF-κB Intensity (a.u)** | 4.5±0.8 | 11.6±1.2 | 4.0±0.5 | 4.4±1.0 | 4.0±0.4 | 10.0±1.3 |

That is, it was confirmed that *Bifidobacterium bifidum* P61, *Lactobacillus paracasei* P62, or a mixture thereof may alleviate inflammation by reducing the expression of inflammatory markers.

## Claims

1. *Bifidobacterium bifidum* P61 KCCM13367P.

2. The *Bifidobacterium bifidum* P61 KCCM13367P of claim 1, wherein the *Bifidobacterium bifidum* P61 KCCM13367P comprises the 16S rDNA nucleotide sequence of SEQ ID NO: 1.

3. *Lactobacillus paracasei* P62 KCCM13368P.

4. The *Lactobacillus paracasei* P62 KCCM13368P of claim 3, wherein the *Lactobacillus paracasei P62* KCCM13368P comprises the 16S rDNA nucleotide sequence of SEQ ID NO: 2.

5. A pharmaceutical composition for preventing or treating sarcopenia, cognitive impairment and inflammatory diseases, comprising *Bifidobacterium bifidum* P61 KCCM13367P, *Lactobacillus paracasei* P62 KCCM13368P, or a mixture thereof.

6. The pharmaceutical composition of claim 5, wherein the sarcopenia is at least one selected from the group consisting of muscular atrophy, disuse atrophy, spinal muscular amyotrophy, dystrophy, muscle rigidity, muscular hypotonia, muscle weakness, muscular dystrophy, amyotrophic lateral sclerosis, spinobulbar muscular atrophy and myasthenia gravis.

7. The pharmaceutical composition of claim 5, wherein the cognitive impairment is at least one selected from the group consisting of Alzheimer's disease, Huntington's disease, vascular dementia, Pick's disease, Parkinson's disease, Creutzfeldt-Jakob disease and dementia.

8. The pharmaceutical composition of claim 5, wherein the inflammatory disease is at least one selected from the group consisting of inflammatory bowel disease (IBD), arthritis, gout, hepatitis, obesity, gastritis, nephritis, diabetes, tuberculosis, bronchitis, pleurisy, peritonitis, spondylitis, pancreatitis, urethritis, cystitis, vaginitis, atherosclerosis, sepsis, and periodontitis.

9. The pharmaceutical composition of claim 5, comprising *Bifidobacterium bifidum* P61 KCCM13367P and *Lactobacillus paracasei* P62 KCCM13368P in a ratio of 10:1 to 1:10 based on colony forming units (CFU).

10. The pharmaceutical composition of claim 9, comprising *Bifidobacterium bifidum* P61 KCCM13367P and *Lactobacillus paracasei* P62 KCCM13368P in a ratio of 1:6 to 1:1 based on colony forming units (CFU).

11. A food composition for preventing or ameliorating sarcopenia, cognitive impairment, and inflammatory diseases, comprising *Bifidobacterium bifidum* P61 KCCM13367P, *Lactobacillus paracasei* P62 KCCM13368P, or a mixture thereof.

12. The food composition of claim 11, wherein the sarcopenia is at least one selected from the group consisting of muscular atrophy, disuse atrophy, spinal muscular atrophy, dystrophy, muscle rigidity, muscular hypotonia, muscle weakness, muscular dystrophy, amyotrophic lateral sclerosis, spinobulbar muscular atrophy, and myasthenia gravis.

13. The food composition of claim 11, wherein the cognitive impairment is at least one selected from the group consisting of Alzheimer's disease, Huntington's disease, vascular dementia, Pick's disease, Parkinson's disease, Creutzfeldt-Jakob disease, and dementia.

14. The food composition of claim 11, wherein the inflammatory disease is at least one selected from the group consisting of inflammatory bowel disease, arthritis, gout, hepatitis, obesity, gastritis, nephritis, diabetes, tuberculosis, bronchitis, pleurisy, peritonitis, spondylitis, pancreatitis, urethritis, cystitis, vaginitis, atherosclerosis, sepsis and periodontitis.

15. The food composition of claim 11, comprising *Bifidobacterium bifidum* P61 KCCM13367P and *Lactobacillus paracasei* P62 KCCM13368P in a ratio of 10:1 to 1:10 based on colony forming units (CFU).

16. The food composition of claim 15, comprising *Bifidobacterium bifidum* P61 KCCM13367P and *Lactobacillus paracasei* P62 KCCM13368P in a ratio of 1:6 to 1:1 based on colony forming units (CFU).

17. A pharmaceutical composition comprising *Bifidobacterium bifidum* P61 KCCM13367P, *Lactobacillus paracasei* P62 KCCM13368P, or a mixture thereof; and a pharmaceutically acceptable carrier.

18. A method for preventing or treating sarcopenia, cognitive impairment, and inflammatory diseases, comprising administering *Bifidobacterium bifidum* P61 KCCM13367P, *Lactobacillus paracasei* P62 KCCM13368P, or a mixture thereof to a subject in need thereof.

19. Use of *Bifidobacterium bifidum* P61 KCCM13367P, *Lactobacillus paracasei* P62 KCCM13368P, or a mixture thereof, in the manufacture of a medicament for the treatment of one or more diseases selected from the group consisting of sarcopenia, cognitive impairment and inflammatory diseases.

20. *Bifidobacterium bifidum* P61 KCCM13367P, *Lactobacillus paracasei* P62 KCCM13368P, or a mixture thereof, for use in the prevention or treatment of one or more diseases selected from the group consisting of sarcopenia, cognitive impairment, and inflammatory diseases.
